# EUROPEAN PATENT SPECIFICATION

(11) **EP 3 562 784 B1**
(45) Date of publication and mention of the grant of the patent: **02.11.2022**
(21) Application number: 17832787.0
(22) Date of filing: 29.12.2017
(51) Int. Cl.: C01C 1/12, B01J 2/00, C01C 1/242, C05C 1/02, C05C 3/00, C07C 273/16

(54) **METHOD FOR PROCESSING A GAS STREAM COMPRISING UREA DUST AND AMMONIA**
VERFAHREN ZUR VERARBEITUNG EINES GASSTROMS MIT HARNSTOFFSTAUB UND AMMONIAK
PROCÉDÉ DE TRAITEMENT D'UN FLUX DE GAZ CONTENANT DE LA POUSSIÈRE D'URÉE ET DE L'AMMONIAC

(30) Priority: 30.12.2016 EP 16207499
(43) Date of publication of application: 06.11.2019
(73) Proprietor: YARA International ASA, 0243 Oslo (NO)
(72) Inventor: VOLKE, Howard, 4541 HJ Sluiskil (NL); VAN BELZEN, Ruud, 4541 HJ Sluiskil (NL); WINNE, Erika, 4541 HJ Sluiskil (NL)
(74) Representative: AWA Sweden AB
(86) International application number: PCT/EP2017/084819
(87) International publication number: WO 2018/122379

(56) References cited:
- CN-A- 101 863 696
- CN-U- 202 933 617
- JP-A- 2000 001 466
- JP-A- 2000 279 736

## Description

### TECHNICAL FIELD

The present invention relates to the field of processing a gas stream comprising urea dust and ammonia.

### INTRODUCTION

The production of urea is known from carbon dioxide and an excess amount of ammonia, and provides a urea granulate, suitable for fertilizer applications. Minor amount of solid urea dust and ammonia (liquid or gaseous) are however entrapped in effluent gas streams. Before disposal into the atmosphere, the amount of urea dust and ammonia is to be reduced to environmentally acceptable levels. Preferably, such urea and ammonia is recycled.

JP 9,227,493 aims to clean such exhaustion gas by simultaneously recovering urea and ammonia from exhaust gas containing urea dust and ammonia gas by bringing the exhaust gas containing urea dust and ammonia gas into contact with an aqueous urea solution, and more preferably an aqueous urea solution with a pH of 3.5 to 5.5 is used.

WO 2011/032786 describes a method for recovery of urea dust and ammonia from a gas stream by contacting said gas stream with an aqueous sulphuric acid solution, thus forming an acidic solution of ammonium sulphate and urea, whereby the acidic solution is neutralized using ammonia and subsequently concentrated to a urea ammonium sulphate melt comprising less than 5 wt.% of water. This melt is then converted into solid particles comprising urea and ammonium sulphate.

JP 2000/001466 provides a method for recovering and utilizing urea dust and ammonia in exhaust gases discharged from a urea granulation unit by passing exhaust gases containing urea dust and ammonia, discharged from a urea granulation unit, to a first scrubbing tower in which an aqueous urea solution is circulated to mainly recover the urea dust, then pass the treated gases to a second scrubbing tower in which acid-added water is circulated to mainly recover ammonia, and mix the recovered solutions with the stock for the urea granulation unit to produce the urea product. Said method is focused on production of urea and is furthermore not concerned with the efficient removal or separation of ammonia from urea.

Methods according to the state of the art are however limited. Contacting ammonia with sulphuric acid in presence of urea provides an inherent risk of decomposition since urea decomposes to carbon dioxide and ammonium sulphate upon contact with sulphuric acid. This decomposition risk is more pronounced since the reaction of ammonia with sulphuric acid is highly exothermic. Therefore, a complete or quantitative removal of ammonia from urea is mandatory for safe production of urea ammonium sulphate. Furthermore, the state of the art provides sequential process schemes which are very rigid and do not allow for large variations in gas stream feed composition as well as in variations of product output. Additionally, such processes do not allow for an extended degree of materials and energy optimization of the entire process.

It will be shown that the present invention provides a process for significantly reducing or completely avoiding decomposition risk, and simultaneously allows for optimization in materials and energy balances.

### SUMMARY OF THE INVENTION

The current invention provides a solution for at least one of the above mentioned problems by providing a method for processing a gas stream comprising at least urea dust and ammonia (liquid or gaseous). The present invention is defined by the appended claims.

The present invention provides a method for processing a gas stream comprising air, urea dust and ammonia, for the production of urea ammonium sulphate, whereby the urea dust and ammonia in said gas stream is separated from each other in a multi-stage separator at a temperature between 60 °C and 70 °C and further processed into a separate urea processing line and an ammonia processing line, respectively, wherein, prior to said separation, said urea dust is sequestered from said gas stream comprising air, urea dust and ammonia by absorption into a water aerosol in a mist chamber, thereby obtaining a urea aerosol, which is subsequently condensed.

In one embodiment, the method for processing a gas stream comprising air, urea dust and ammonia, comprises the steps of:
(a) separating said ammonia from said urea aerosol,
(b) converting said urea aerosol into an aqueous urea solution,
(c) converting said ammonia to an aqueous ammonium sulphate solution, and
(d) mixing said aqueous urea solution and said aqueous ammonium sulphate solution, thereby obtaining an urea ammonium sulphate solution.

The gas stream comprising urea dust and ammonia further comprises air. Hence the gas stream, as used in this application, is a stream of at least air, comprising at least nitrogen and oxygen in variable concentration. Preferably, the air is atmospheric air comprising a naturally occurring nitrogen and oxygen content, preferably comprising about 78 % nitrogen and about 21 % oxygen, more preferably comprising about 78 % nitrogen, about 21 % oxygen, about 0.9 % argon, about 0.04 % carbon dioxide, and small amounts of other gases.

The present invention is advantageous in that (i) sulphuric acid used for converting ammonia to ammonium sulphate does not contact with urea, thereby avoiding decomposition risk upon urea decomposition; (ii) urea and ammonium sulphate are processed separately, allowing for improved process control and energy balance; (iii) the proposed method allows to adapt sulphur (S) to nitrogen (N) (S/N) ratio of the ammonium sulphate solution to be optimized independently from the S/N ratio of the urea ammonium sulphate.

### DESCRI PTI ON OF THE FI GURES

By means of further guidance, a figure is included to better appreciate the teaching of the present invention. Said figure is intended to assist the description of the invention and is nowhere intended as a limitation of the presently disclosed invention.

The figure and symbols contained therein have the meaning as commonly understood by one of ordinary skill in the art to which this invention belongs.

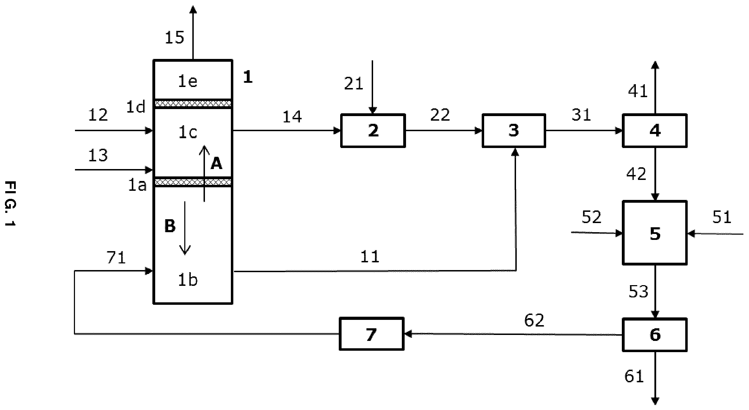

Figure 1 essentially shows that a gas feed of air, urea dust and ammonia is separated over multiple separation sections of a scrubber to provide a separate urea processing line and a separate ammonia processing line.

### DETAILED DESCRI PTI ON OF THE I NVENTI ON

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints. All percentages are to be understood as percentage by weight and are abbreviated as "%wt.", unless otherwise defined or unless a different meaning is obvious to the person skilled in the art from its use and in the context wherein it is used.

The term "S/N ratio" as used within the context of the present disclosure is to be understood as the ratio of sulphur (S) atoms to nitrogen (N) atoms within a mixture, i.e. a urea ammonium sulphate mixture.

The current invention provides in a solution for at least one of the above mentioned problems by providing a method for processing a gas stream comprising urea dust and ammonia.

In a first aspect, the present invention provides a method for processing a gas stream comprising air, urea dust and ammonia, for the production of urea ammonium sulphate, whereby the urea dust and ammonia in said gas stream is separated from each other in a multi-stage separator at a temperature between 60 °C and 70 °C and further processed into a separate urea processing line and an ammonia processing line, respectively, wherein, prior to said separation, said urea dust is sequestered from said gas stream comprising air, urea dust and ammonia by absorption into a water aerosol in a mist chamber, thereby obtaining a urea aerosol, which is subsequently condensed.

In an embodiment, the methodof the present invention further comprises the steps of:
(a) separating said ammonia from said urea aerosol),
(b) converting said urea aerosol) into an aqueous urea solution,
(c) converting said ammonia into an aqueous ammonium sulphate solution,
(d) mixing said aqueous urea solution and said aqueous ammonium sulphate solution, thereby obtaining a urea ammonium sulphate solution, and
(e) optionally, concentrating said aqueous urea solution, said aqueous ammonium sulphate solution, and/or said urea ammonium sulphate solution. A first advantage of the present invention is that sulphuric acid used for converting ammonia to ammonium sulphate does not contact with urea. Urea and sulphuric acid can react, resulting in the decomposition of urea to ammonia and carbon dioxide, after which the ammonia can react with the sulphuric acid. Such a reaction is very exothermic and the evolution of carbon dioxide gas during the reaction may result in decomposition risk.

A second advantage of the present invention is that urea and ammonium sulphate are processed separately, allowing for the respective urea solution and ammonium sulphate solution to be mixed in the required S/N ratio. This is advantageous since other process parameters such as rate of adding sulphuric acid, evaporation of water and temperature control during preceding steps can remain constant during the previous process steps. This significantly allows for a better process control, avoids further decomposition risks and allows for a better energy balance of the process.

A third advantage of the present invention is that the proposed method allows to adapt S/N ratio of the ammonium sulphate solution to be optimized for minimal corrosive behaviour, while the S/N ratio of the urea ammonium sulphate solution can be optimized independently to provide a desired fertilizer having a predetermined S/N ratio.

In a preferred embodiment, the present invention provides a method according to the first aspect, whereby said ammonia is separated from said urea dust by passage of said gas stream comprising air, urea dust and ammonia over a multi-stage separation scrubber. A gas and dust multi-stage separation scrubber allows for a selective separation of ammonia and urea dust.

In a preferred embodiment, the present invention provides a method according to the first aspect, whereby ammonia separated from said urea dust is subsequently contacted with an aqueous solution of sulphuric acid, thereby obtaining an aqueous solution of ammonium sulphate and an excess of sulphuric acid. Contacting of ammonia with sulphuric acid preferably occurs after passing a wet packing system, which is kept wet by adding water and which is sprayed upon with an aqueous sulphuric acid solution through a nozzle, thereby providing an acidic film on the packing which increases the contact with the gaseous ammonia. Accordingly, the ammonia can be absorbed effectively. Preferably, the aqueous sulphuric acid has a concentration of at least 50 wt.% of sulphuric acid, and more preferably of at least 90 wt.% of sulphuric acid, and even more preferably of 90, 92, 94, 96, 98 wt.% of sulphuric acid, or any concentration there in between. Lower concentrations of sulphuric acid behave relatively less corrosive, thereby lowering the oxidation resistant requirements towards the materials, i.e. the nozzles and the scrubber. However, when concentration of sulphuric acid become too low, the obtained aqueous solution of ammonium sulphate and an excess of sulphuric acid becomes very diluted, thereby requiring high energy input for the final water content reduction.

In a more preferred embodiment, said excess of sulphuric acid is provided in a molar ratio of sulphuric acid to ammonia smaller than 1.5, and more preferably smaller than 1.1. Larger excesses of sulphuric acid allow for a faster and/or more quantitative removal of ammonia from the gas stream, but also lead to higher excesses of sulphuric acid in the aqueous solution of ammonium sulphate and an excess of sulphuric acid. The excess of sulphuric acid can later be neutralized, i.e. by addition of gaseous or liquid ammonia.

Air and/or other gases in the ammonia gas stream may be evacuated from the scrubber after contact with said aqueous solution of sulphuric acid. In a preferred embodiment, said evacuated gas stream is further at least partially recycled to the compartment of the scrubber for additional contacting with an said aqueous solution of sulphuric acid. This is advantageous for further lowering the concentration of ammonia in the effluent gas which is evacuated from the scrubber. Such effluent gas may be disposed in the atmosphere or may be further treated to additionally lower the concentration of ammonia.

In a preferred embodiment, the present invention provides a method according to the first aspect, whereby said excess of sulphuric acid is neutralized with ammonia to a S/N ratio of about 1:2, and subsequently concentrated to obtain an ammonium sulphate solution. Providing an ammonium sulphate melt in the above mention S/N ratio assures that the melt shows a minimal corrosive behaviour which reduces requirements towards the process equipment.

In a preferred embodiment, the present invention provides a method according to the first aspect, whereby said urea dust in the step of separating urea dust and ammonia from each other is sequestered from said gas stream comprising urea dust and ammonia by absorption in water, thereby obtaining an aqueous solution of urea. Preferably, water is sprayed through a nozzle thereby forming a water vapour in which the urea dust can be entrapped and subsequently condensed to form an aqueous urea solution. As such, urea dust can be effectively removed from the gas stream.

In a preferred embodiment, the present invention provides a method according to the first aspect, whereby said aqueous solution of urea is evaporated to yield urea solid.

In a preferred embodiment, the present invention provides a method according to the first aspect, whereby said aqueous solution of urea is evaporated to yield urea solution having a water content of less than 5 wt.%. More preferably, said urea solution is concentrated to a water content of less than 4 wt.%, and even more preferably less than 3 wt.%.

In a preferred embodiment, the present invention provides a method according to the first aspect, whereby said ammonium sulphate solution and said urea solution are mixed to obtain urea ammonium sulphate having a predetermined S/N ratio.

In a preferred embodiment, the present invention provides a method according to the first aspect, whereby said ammonium sulphate solution and said urea solution are mixed and subsequently granulated to obtain urea ammonium sulphate granules. The proposed granulation of ammonium sulphate solution and urea solution allows for intimately mixing of both product streams to provide a homogeneous mixture of urea ammonium sulphate.

In a preferred embodiment, the present invention provides a method according to the first aspect, whereby water is evaporated from said ammonium sulphate solution and/or from said urea solution before mixing and/or before granulating.

In a preferred embodiment, the present invention provides a method according to the first aspect, whereby said gas stream comprising urea dust and ammonia is an exhaust gas from a urea plant, a urea granulation unit, a urea prilling tower or a chemical fertilizer plant.

In one embodiment of the method for processing a gas stream comprising air, urea dust and ammonia for the production of urea ammonium sulphate, said gas stream is contacted with water in the multi-stage separator, thereby obtaining a gas phase comprising ammonia and a urea solution, and said method further comprises the steps of:
(i) contacting said gas phase comprising ammonia with an excess of sulphuric acid in water, thereby obtaining an aqueous solution of ammonium sulphate and an excess of sulphuric acid,
(ii) neutralizing said aqueous solution of ammonium sulphate and an excess of sulphuric acid with ammonia, thereby obtaining an ammonium sulphate solution,
(iii) mixing said urea solution with said ammonium sulphate solution,
(iv) concentrating the mixed urea and ammonium sulphate solution,
(v) adding ammonium sulphate and/or urea to the mixed urea ammonium sulphate solution, thereby increasing the ammonium sulphate concentration to a desired concentration and capacity,
(vi) granulating the mixture obtained from step (v), thereby obtaining solid particles comprising urea ammonium sulphate.

The process of the invention is illustrated more detailed in Figure 1. Figure 1 essentially shows that a gas feed comprising air, urea dust and ammonia is separated over a packing to provide a urea processing line and an ammonia processing line.

A supply flow **62** comprising air, urea dust, ammonia and optionally a smaller fraction of ammonium sulphate, is contacted with a water mist in mist chamber **7.** The mist chamber **7** ensures that urea dust is absorbed in water droplets, thereby forming an urea aerosol. The accordingly formed urea aerosol **71** is led to a multi-stage separator **1** at a temperature between 60°C and 70°C. At such temperatures, ammonia in the aerosol **71** is predominantly in the gaseous phase, and as such can pass over the packing **1a** according to arrow A. To improve separation of urea dust and ammonia, it is ensured that packing **1a** is kept wet by addition of water via inlet **13.** Urea is trapped in the compartment **1b** of the separator before the packing **1a** and condensed to a concentrated aqueous urea solution **11**. This concentrated urea solution can then be further concentrated before being mixed with ammonium sulphate in mixer **3.**

The ammonia flow which has passed the packing **1a** is subsequently scrubbed with a 96 wt.% sulphuric acid solution **12** in the top part **1c** of the scrubber **1,** thereby obtaining an acidic ammonium sulphate solution **14.** In order to sequester ammonia in the sulphuric acid solution **12,** the solution is mixed intensively to improve contact between the gaseous ammonia and liquid sulphuric acid. Suitable scrubbers can be selected from any of the wet-type scrubbers well known in the industry. Scrubbers may be selected from the type of scrubbers as summarized in Chemical Engineers Handbook (Perry and Chilton), fifth edition, page 20-94 to 20-103. Circulation of the acidic ammonium sulphate solution over the top part **1e** of the scrubber **1** may be advisable for a more quantitative removal of ammonia from the exhaust air stream **15.** Exhaust air **15** is evacuated from the reactor **1** and disposed in the atmosphere or - if necessary - further treated to meet environmental regulations.

The acidic ammonium sulphate solution is transferred through line **14** to reactor **2** and neutralized using ammonia, added through line **21.** The exothermic reaction produces heat which allows water in the solution to be heated. Accordingly, an aqueous ammonium sulphate solution with an S/N ratio of 1:2 is transferred through line **22** to a mixer **3** where it is mixed with the urea solution form line **11** coming from scrubber **1.** The mixed solution is fed through line **31** to an evaporator **4** for evaporating water, which is evacuated through line **41** and for providing an ammonium sulphate melt comprising less than 5 wt.% of water.

The concentrated urea ammonium sulphate solution form separator **4** can be mixed with ammonium sulphate **51** and/or a concentrated urea solution **52** in mixer **5** to ensure the proper S/N ratio of the final product **53.** As such, the concentration of the urea ammonium sulphate solution or slurry (in line **53** to the granulator **6)** can be controlled by adjusting the ammonium sulphate feed (through line **51**) and the urea feed (through line **52)** in amounts to provide a desired, predetermined S/N ratio and granulated in a granulation unit **6** to provide urea ammonium sulphate granules, which are discharged through line **61.** Alternatively, this process can be carried out in a prilling tower or a pelletizer in series with a solidification belt.

### List of reference numbers

- 1: Scrubber, multi-stage separator
- 1a: Urea packing, multi-stage aerosol separator
- 1b: Compartment of scrubber
- 1c: Compartment of scrubber
- 1d: Ammonia packing
- 1e: Scrubber top compartment
- 2: Reactor Evaporator
- 3: Mixer
- 4: Evaporator/separator
- 5: Mixer
- 6: Granulation unit
- 7: Aerosol chamber/mist chamber (demister)
- 11: Urea solution
- 12: Sulphuric acid
- 13: Water
- 14: Acidic ammonium sulphate solution
- 15: Exhaust air stream
- 21: Ammonia supply
- 22: Ammonium sulphate solution
- 31: Urea ammonium sulphate solution
- 41: Water vapour
- 42: Highly concentrated ammonium sulphate solution
- 51: Ammonium sulphate
- 52: Concentrated urea solution
- 53: Highly concentrated urea ammonium sulphate solution or slurry
- 61: Discharge flow end product
- 62: Air, dust and ammonia from granulation unit
- 71: Urea aerosol comprising air and ammonia

## Claims

1. Method for processing a gas stream comprising air, urea dust and ammonia, for the production of urea ammonium sulphate, whereby the urea dust and ammonia in said gas stream is separated from each other in a multi-stage separator at a temperature between 60 °C and 70 °C and further processed into a separate urea processing line and an ammonia processing line, respectively, **characterized in that,** prior to said separation, said urea dust is sequestered from said gas stream comprising air, urea dust and ammonia by absorption into a water aerosol in a mist chamber, thereby obtaining a urea aerosol, which is subsequently condensed.

2. Method according to claim 1, comprising the further steps of:
(a) separating said ammonia from said urea aerosol,
(b) converting said urea aerosol into an aqueous urea solution,
(c) converting said ammonia into an aqueous ammonium sulphate solution,
(d) mixing said aqueous urea solution and said aqueous ammonium sulphate solution, thereby obtaining a urea ammonium sulphate solution, and
(e) optionally, concentrating said aqueous urea solution, said aqueous ammonium sulphate solution, and/or said urea ammonium sulphate solution.

3. Method according to claim 1, whereby ammonia, separated from said urea dust, is subsequently contacted with an aqueous solution of sulphuric acid, thereby obtaining an aqueous solution of ammonium sulphate and an excess of sulphuric acid.

4. Method according to claim 3, whereby said excess of sulphuric acid is neutralized with ammonia to a ratio of sulphur (S) atoms to nitrogen (N) atoms (S/N ratio) of about 1:2, and subsequently concentrated to obtain an ammonium sulphate solution.

5. Method according to claim 4, whereby said aqueous urea solution and said ammonium sulphate solution are mixed, thereby obtaining a urea ammonium sulphate solution.

6. Method according to claim 5, whereby said aqueous urea ammonium sulphate solution is concentrated to a water content of less than 5 wt%.

7. Method according to claim 5 or 6, whereby said urea ammonium sulphate solution is mixed with urea and/or ammonium sulphate to obtain an urea ammonium sulphate solution or slurry having a predetermined ratio of sulphur (S) atoms to nitrogen (N) atoms (S/N ratio).

8. Method according to claim 7, whereby said urea ammonium sulphate solution or slurry is granulated to obtain urea ammonium sulphate granules.

9. Method according to any one of claims 1 to 8, whereby said gas stream comprising urea dust and ammonia is an exhaust gas from a plant, a granulation unit, a prilling tower or a chemical fertilizer plant.

10. Method according to claim 1, wherein in the multi-stage separator said gas stream is contacted with water, thereby obtaining a gas phase comprising ammonia and a urea solution, and said method further comprising the steps of:
(i) contacting said gas phase comprising ammonia with an excess of sulphuric acid in water, thereby obtaining an aqueous solution of ammonium sulphate and an excess of sulphuric acid,
(ii) neutralizing said aqueous solution of ammonium sulphate and an excess of sulphuric acid with ammonia, thereby obtaining an ammonium sulphate solution,
(iii) mixing said urea solution with said ammonium sulphate solution,
(iv) concentrating the mixed urea and ammonium sulphate solution,
(v) adding ammonium sulphate and/or urea to the mixed urea ammonium sulphate solution, thereby increasing the ammonium sulphate concentration to a desired concentration and capacity,
(vi) granulating the mixture obtained from step (v), thereby obtaining solid particles comprising urea ammonium sulphate.

## Patentansprüche

1. Verfahren zur Verarbeitung eines Gasstroms, der Luft, Harnstoffstaub und Ammoniak umfasst, für die Herstellung von Harnstoff-Ammoniumsulfat, wobei der Harnstoffstaub und der Ammoniak in dem Gasstrom in einem mehrstufigen Separator bei einer Temperatur von zwischen 60 °C und 70 °C voneinander getrennt und in einer getrennten Harnstoff-Verarbeitungslinie bzw. einer Ammoniak-Verarbeitungslinie weiter verarbeitet werden, **dadurch gekennzeichnet, dass** vor dem Trennen der Harnstoffstaub durch Absorption in ein Wasseraerosol in einer Nebelkammer von dem Gasstrom, der Luft, Harnstoffstaub und Ammoniak umfasst, sequestriert wird, um ein Harnstoffaerosol zu erhalten, das anschließend kondensiert wird.

2. Verfahren gemäß Anspruch 1, umfassend die weiteren Schritte:
(a) Abtrennen des Ammoniaks von dem Harnstoffaerosol,
(b) Umwandeln des Harnstoffaerosols zu einer wässrigen Harnstofflösung,
(c) Umwandeln des Ammoniaks zu einer wässrigen Ammoniumsulfatlösung,
(d) Mischen der wässrigen Harnstofflösung und der wässrigen Ammoniumsulfatlösung, um eine Harnstoff-Ammoniumsulfat-Lösung zu erhalten, und
(e) gegebenenfalls Konzentrieren der wässrigen Harnstofflösung, der wässrigen Ammoniumsulfatlösung und/oder der Harnstoff-Ammoniumsulfat-Lösung.

3. Verfahren gemäß Anspruch 1, wobei von dem Harnstoffstaub abgetrennter Ammoniak anschließend mit einer wässrigen Lösung von Schwefelsäure in Kontakt gebracht wird, um eine wässrige Lösung von Ammoniumsulfat und einen Überschuss von Schwefelsäure zu erhalten.

4. Verfahren gemäß Anspruch 3, wobei der Überschuss von Schwefelsäure mit Ammoniak auf ein Verhältnis von Schwefel(S)-Atomen zu Stickstoff(N)-Atomen (S/N-Verhältnis) von etwa 1:2 neutralisiert wird und anschließend konzentriert wird, um eine Ammoniumsulfatlösung zu erhalten.

5. Verfahren gemäß Anspruch 4, wobei die wässrige Harnstofflösung und die Ammoniumsulfatlösung gemischt werden, um eine Harnstoff-Ammoniumsulfat-Lösung zu erhalten.

6. Verfahren gemäß Anspruch 5, wobei die wässrige Harnstoff-Ammoniumsulfat-Lösung auf einen Wassergehalt von weniger als 5 Gew.-% konzentriert wird.

7. Verfahren gemäß Anspruch 5 oder 6, wobei die Harnstoff-Ammoniumsulfat-Lösung mit Harnstoff und/oder Ammoniumsulfat gemischt wird, um eine Harnstoff-Ammoniumsulfat-Lösung oder -aufschlämmung mit einem vorgegebenen Verhältnis von Schwefel(S)-Atomen zu Stickstoff(N)-Atomen (S/N-Verhältnis) zu erhalten.

8. Verfahren gemäß Anspruch 7, wobei die Harnstoff-Ammoniumsulfat-Lösung oder -aufschlämmung granuliert wird, um Harnstoff-Ammoniumsulfat-Granulat zu erhalten.

9. Verfahren gemäß einem der Ansprüche 1 bis 8, wobei der Gasstrom, der Harnstoffstaub und Ammoniak umfasst, ein Abgas von einer Anlage, einer Granulationseinheit, einem Prillturm oder einer Kunstdüngeranlage ist.

10. Verfahren gemäß Anspruch 1, wobei
in dem mehrstufigen Separator der Gasstrom mit Wasser in Kontakt gebracht wird, um eine Gasphase zu erhalten, die Ammoniak und eine Harnstofflösung umfasst, und das Verfahren ferner die Schritte umfasst:
(i) Inkontaktbringen der Gasphase, die Ammoniak umfasst, mit einem Überschuss von Schwefelsäure in Wasser, um eine wässrige Lösung von Ammoniumsulfat und einen Überschuss von Schwefelsäure zu erhalten,
(ii) Neutralisieren der wässrigen Lösung von Ammoniumsulfat und eines Überschusses von Schwefelsäure mit Ammoniak, um eine Ammoniumsulfatlösung zu erhalten,
(iii) Mischen der Harnstofflösung mit der Ammoniumsulfatlösung,
(iv) Konzentrieren der Harnstoff- und Ammoniumsulfat-Mischlösung,
(v) Zugeben von Ammoniumsulfat und/oder Harnstoff zu der Harnstoff-Ammoniumsulfat-Mischlösung, um die Konzentration des Ammoniumsulfats auf eine gewünschte Konzentration und Kapazität zu erhöhen,
(vi) Granulieren des bei Schritt (v) erhaltenen Gemischs, um feste Partikel zu erhalten, die Harnstoff-Ammoniumsulfat umfassen.

## Revendications

1. Procédé de traitement d'un flux de gaz comprenant de l'air, de la poussière d'urée et de l'ammoniac, pour la production d'urée-sulfate d'ammonium, dans lequel la poussière d'urée et l'ammoniac dans ledit flux de gaz sont séparés l'un de l'autre dans un séparateur à étages multiples à une température comprise entre 60 °C et 70 °C et ensuite traités dans une ligne de traitement d'urée séparée et une ligne de traitement d'ammoniac, respectivement, **caractérisé en ce que,** avant ladite séparation, ladite poussière d'urée est piégée à partir dudit flux de gaz comprenant de l'air, de la poussière d'urée et de l'ammoniac par absorption dans un aérosol d'eau dans une chambre à brouillard, de façon à obtenir un aérosol d'urée, qui est ensuite condensé.

2. Procédé selon la revendication 1, comprenant les étapes supplémentaires de :
(a) séparation dudit ammoniac dudit aérosol d'urée,
(b) conversion dudit aérosol d'urée en une solution aqueuse d'urée,
(c) conversion dudit ammoniac en une solution aqueuse de sulfate d'ammonium,
(d) mélange de ladite solution aqueuse d'urée et ladite solution aqueuse de sulfate d'ammonium,
de façon à obtenir une solution d'urée-sulfate d'ammonium, et
(e) facultativement, concentration de ladite solution aqueuse d'urée, ladite solution aqueuse de sulfate d'ammonium et/ou ladite solution d'urée-sulfate d'ammonium.

3. Procédé selon la revendication 1, dans lequel l'ammoniac, séparé de ladite poussière d'urée, est ensuite mis en contact avec une solution aqueuse d'acide sulfurique, de façon à obtenir une solution aqueuse de sulfate d'ammonium et un excès d'acide sulfurique.

4. Procédé selon la revendication 3, dans lequel ledit excès d'acide sulfurique est neutralisé avec de l'ammoniac à un rapport des atomes de soufre (S) aux atomes d'azote (N) (rapport S/N) d'environ 1:2, et ensuite concentré pour obtenir une solution de sulfate d'ammonium.

5. Procédé selon la revendication 4, dans lequel ladite solution aqueuse d'urée et ladite solution de sulfate d'ammonium sont mélangées, de façon à obtenir une solution d'urée-sulfate d'ammonium.

6. Procédé selon la revendication 5, dans lequel ladite solution aqueuse d'urée-sulfate d'ammonium est concentrée à une teneur en eau inférieure à 5 % en poids.

7. Procédé selon la revendication 5 ou 6, dans lequel ladite solution d'urée-sulfate d'ammonium est mélangée avec de l'urée et/ou du sulfate d'ammonium pour obtenir une solution ou une suspension concentrée d'urée-sulfate d'ammonium ayant un rapport prédéterminé des atomes de soufre (S) aux atomes d'azote (N) (rapport S/N).

8. Procédé selon la revendication 7, dans lequel ladite solution ou suspension concentrée d'urée-sulfate d'ammonium est granulée pour obtenir des granulés d'urée-sulfate d'ammonium.

9. Procédé selon l'une quelconque des revendications 1 à 8, dans lequel ledit flux de gaz comprenant de la poussière d'urée et de l'ammoniac est un gaz d'échappement provenant d'une installation, d'une unité de granulation, d'une tour de grelonage ou d'une installation de fabrication d'engrais chimiques.

10. Procédé selon la revendication 1, dans lequel dans le séparateur à étages multiples, ledit flux de gaz est mis en contact avec de l'eau, de façon à obtenir une phase gazeuse comprenant ammoniac et a urée solution, et ledit procédé comprenant en outre les étapes de :
(i) mise en contact de ladite phase gazeuse comprenant de l'ammoniac avec un excès d'acide sulfurique dans de l'eau, de façon à obtenir une solution aqueuse de sulfate d'ammonium et un excès d'acide sulfurique,
(ii) neutralisation de ladite solution aqueuse de sulfate d'ammonium et d'un excès d'acide sulfurique avec de l'ammoniac, de façon à obtenir une solution de sulfate d'ammonium,
(iii) mélange de ladite solution d'urée avec ladite solution de sulfate d'ammonium,
(iv) concentration de la solution mixte d'urée et de sulfate d'ammonium,
(v) ajout de sulfate d'ammonium et/ou d'urée à la solution mixte d'urée-sulfate d'ammonium, de façon à augmenter la concentration de sulfate d'ammonium à une concentration et une capacité souhaitées,
(vi) granulation du mélange obtenu à partir de l'étape (v), de façon à obtenir des particules solides comprenant de l'urée-sulfate d'ammonium.
